# EUROPEAN PATENT SPECIFICATION

(11) **EP 2 457 098 B1**
(45) Date of publication and mention of the grant of the patent: **13.08.2014**
(21) Application number: 10737701.2
(22) Date of filing: 16.07.2010
(51) Int. Cl.: G01N 33/92

(54) **METHOD FOR PREDICTING WEIGHT GAIN ASSOCIATED WITH A PHARMACEUTICAL THERAPY**
VERFAHREN ZUR VORHERSAGE DER MIT EINER PHARMAZEUTISCHEN THERAPIE VERBUNDENEN GEWICHTSZUNAHME
MÉTHODE DE PRÉVISION DU GAIN DE POIDS ASSOCIÉ AVEC UN TRAITEMENT PHARMACEUTIQUE

(30) Priority: 22.07.2009 US 271508 P
(43) Date of publication of application: 30.05.2012
(73) Proprietor: Eli Lilly and Company, Indianapolis, IN 46285 (US)
(72) Inventor: LEOHR, Jennifer, Kay, Indianapolis, Indiana 46285 (US)
(74) Representative: Suarez-Miles, Ana Sanchiz
(86) International application number: PCT/US2010/042208
(87) International publication number: WO 2011/011267

(56) References cited:
- WO-A1-2009/085917
- WO-A1-2009/085927
- WO-A2-2007/050318

## Description

The literature has reported an observation of treatment emergent weight gain in subpopulations of the patients treated with several current antipsychotics, antidepressants, mood stabilizers, and other psychological disorders, as well as some treatments for glycemic control such as PPAR-gamma agonists. WO 2007/050318 A2 discloses lipidomies to evaluate side effects to a treatment. To date, there are no methods for determining prior to observation of the actual weight gain which individual patients will be susceptible to treatment emergent weight gain and which will not. Such a method would be of great value in prescreening prospective patients to better inform therapy decisions.

The present disclosure provides a biomarker and methods, for using the biomarker for predicting whether an individual patient will be subject to treatment emergent weight gain with a given course of pharmaceutical therapy, as for example treatment with an atypical antipsychotic, as for example olanzapine and the like.

In one aspect, it has been found that changes in the concentration of large triglyceride rich lipoprotein particles of the V6 subclass (TRL V6) can be used as a biomarker for the modulation of triglyceride and/or lipoprotein metabolism in a mammal induced by a pharmaceutical agent. Specifically, a pharmaceutical agent having an observed treatment emergent weight gain in a subpopulation of patients, as for example atypical antipsychotics, as for example 2-methyl-4-(4-methyl-1-piperazinyl)-10H-thieno[2,3-b][1,5]benzodiazepine or a salt thereof (olanzapine), will produce an increase in TRL V6 concentration in patients who are susceptible to treatment emergent weight gain. Knowing this degree of risk on a patient by patient basis prior to beginning treatment or early in a course of treatment can be used to better inform treatment options and/or indicate co-treatments to mitigate potential weight gain.

As such, one embodiment of the present invention provides a method of determining a human patient's risk of treatment emergent weight gain during treatment with a pharmaceutical agent, comprising determining in a biosample isolated from the patient whether there is an increase in TRL V6 concentration in response to one or more doses of the pharmaceutical agent wherein a significant increase in TRL V6 concentration indicates the patient is at risk of treatment emergent weight gain during treatment with the pharmaceutical agent.

In another example, there is provided a method for determining in a human patient the risk of treatment emergent weight gain during treatment with a pharmaceutical agent comprising the steps of:
1) administering to the patient a dose of the pharmaceutical agent;
2) measuring the patient's TRL V6 response to the dose;
wherein a significant TRL V6 response indicates the patient is at risk of treatment emergent weight gain during treatment with the pharmaceutical agent.

In another example, there is provided a method for determining in a human patient the risk of treatment emergent weight gain during treatment with a pharmaceutical agent comprising the steps of:
1) administering to the patient a dose of the pharmaceutical agent wherein the patient is in a fasted state;
2) measuring the patient's TRL V6 response to the dose;
wherein a significant TRL V6 response indicates the patient is at risk of treatment emergent weight gain during treatment with the pharmaceutical agent.

In another aspect, there is provided a method for determining a human patient's risk of treatment emergent weight gain during treatment with a pharmaceutical agent comprising the steps of:
1) administering to the patient a first fat load;
2) measuring the patient's TRL V6 response to the first fat load;
3) administering to the patient a dose of the pharmaceutical agent in conjunction with administering to the patient a second fat load;
4) measuring the patient's TRL V6 response to the second fat load;
5) determining whether there is an increase in TRL V6 response to the second fat load compared to the TRL V6 response to the first fat load;
wherein a significant increase in TRL V6 response indicates the patient is at risk of treatment emergent weight gain during treatment with the pharmaceutical.

In a further example, there is provided a method for determining a human patient's risk of treatment emergent weight gain during treatment with a pharmaceutical agent comprising the steps of:
1) administering to the patient a dose of the pharmaceutical agent in conjunction with administering to the patient a fat load;
2) measuring the patient's TRL V6 response to the fat load;
3) determining whether there is an increase in TRL V6 response to the fat load compared to a standard TRL V6 response to the fat load absent the pharmaceutical agent;
wherein a significant increase in TRL V6 response indicates the patient is at risk of treatment emergent weight gain during treatment with the pharmaceutical.

In another aspect of the invention, there is provided a method of determining a human patient's risk of treatment emergent weight gain during treatment with a pharmaceutical agent comprising determining in a biosample isolated from the patient whether there is an increase in TRL V6 concentration in response to one or more doses of the pharmaceutical agent, wherein a significant increase in TRL V6 concentration indicates the patient is at risk of treatment emergent weight gain during treatment with the pharmaceutical agent.

In another embodiment of this aspect of the invention, there is provided a method of determining a human patient's risk of treatment emergent weight gain during treatment with a pharmaceutical agent comprising:
measuring the TRL V6 response to one or more doses of the pharmaceutical agent in a biosample isolated from the patient administered said one or more doses of the pharmaceutical agent;
wherein a significant TRL V6 response indicates the patient is at risk of treatment emergent weight gain during treatment with the pharmaceutical agent.

In another embodiment of this aspect of the invention, there is also provided a method of determining a human patient's risk of treatment emergent weight gain during treatment with a pharmaceutical agent comprising the steps of:
1) measuring the TRL V6 response to a first fat load in a biosample isolated from the patient administered the first fat load;
2) measuring the TRL V6 response to a second fat load in a biosample isolated from the patient administered a dose of the pharmaceutical agent in conjunction with administration of the second fat load;
3) determining whether there is an increase in TRL V6 response to the second fat load compared to the TRL V6 response to the first fat load;
wherein a significant increase in TRL V6 response indicates the patient is at risk of treatment emergent weight gain during treatment with the pharmaceutical.

In yet another embodiment of this aspect of the invention, there is provided a method for determining a human patient's risk of treatment emergent weight gain during treatment with a pharmaceutical agent comprising the steps of:
1) measuring the TRL V6 response to a fat load in a biosample isolated from the patient administered a dose of the pharmaceutical agent in conjunction with administration of the fat load;
2) determining whether there is an increase in TRL V6 response to the fat load compared to a standard TRL V6 response to the fat load absent the pharmaceutical agent;
wherein a significant increase in TRL V6 response indicates the patient is at risk of treatment emergent weight gain during treatment with the pharmaceutical.

In particular embodiments of the above methods of the invention, the pharmaceutical agent is an atypical antipsychotic drug. In specific embodiments of the above methods of the invention, the pharmaceutical agent is olanzapine.

In further particular examples, the pharmaceutical agent is a PPAR gamma agonist, as for example a thiazolidinedione. In specific examples of the above methods, the pharmaceutical agent is pioglitazone, rosiglitazone, or troglitazone.

In another aspect of the disclosure, there is provided a system for assessing a patient's risk of treatment emergent weight gain during treatment with a pharmaceutical agent, comprising:
an NMR spectrometer; and
at least one processor in communication with the NMR spectrometer,
wherein the at least one processor is configured to evaluate the NMR signal generated by the NMR spectrometer to determine the TRL V6 concentration, or equivalent, of one or *more in vitro* biosamples from the patient, and to determine whether there is TRL V6 response to the pharmaceutical agent, wherein a significant TRL V6 response indicates the patient is at risk of treatment emergent weight gain during treatment with the pharmaceutical agent.

### Brief Description of the Figures

**Figure** 1 is a graph of lipid methyl group NMR signals for exemplary triglyceride rich lipoprotein particle subfractions (subclasses).
**Figure 2** is a schematic illustration of an NMR system.
**Figure 3** is a schematic diagram of an exemplary data processing system.

In the drawings, like numbers refer to like elements throughout, and thickness, size and dimensions of some components, lines, or features may be exaggerated for clarity. The order of operations and/or steps illustrated in the figures or recited in the claims are not intended to be limited to the order presented unless stated otherwise. Broken lines in the figures, where used, indicate that the feature, operation or step so indicated is optional unless specifically stated otherwise.

For the purposes of this application, the following terms will have the following meanings unless specifically stated otherwise:

The term "fasted state" means the physiological state of an individual after a period without food or calorie containing beverages. In this state, the individual's gastric emptying has been cleared and triglycerides are at a basal level. Time needed to attain a fasted state will vary from individual to individual and on the nature of their prior caloric intake. Typically a fasted state is achieved after fasting for about 5 - 10 hours, preferably between about 6 to 9 hours, as for example about 8 hours.

The term "fat load" means a dose of lipid sufficient to induce hyperlipidemia in a test subject and can take the form of an oral fat load, a fat-containing intravenous infusion, a high-fat foodstuff, a meal, a high fat beverage, or the like. It is also contemplated that a pharmaceutical agent may be developed and used to deliver the fat-load or a simulated fat-load.

The term "TRL V6" refers to TRL (triglyceride rich lipoprotein) particles or subfractions having a diameter between about 90 nm up to as much as about 170 nm, more typically having diameters between about 100-140 nm. The term "TRL V6" can also be defined with respect to the lipid methyl group NMR signal chemical shifts (ppm) corresponding to the estimated diameters as provided in **Table I** below.

The term "TRL V5" refers to large TRL particles having a diameter of between about 60 nm and about 80 nm (see **Table 1** below for the associated NMR chemical shifts).

The term "chylomicron" refers to very large TRL particles having diameters that are larger than TRL V6. As such chylomicrons refers to TRL particles or sub-fractions having a diameter between from about 170 nm up to about 260 nm (see **Table** I below for their associated NMR chemical shifts). It is important to note that there is not a clear demarcation between TRL V5 and TRL V6 nor between TRL V6 and chylomicrons, such that there is a distribution of particle sizes for each subgroup that overlaps in the range between about 80-90 nm for TRL V5-6 and between about 140-170 nm for TRL V6 & chylomicrons.

The term "TRL V6 response" means the increase in TRL V6 triglyceride concentration and/or particle number in a patient or test mammal compared to a baseline concentration and/or particle number. It is to be understood that under some circumstances, it may be advantageous to measure "L TRL ", which for the purposes of this application is taken to mean a subgroup of very large TRL that contains both the TRL V6 and the TRL V5 particle subtypes, as a surrogate to measuring TRL V6 concentrations alone, in that under some circumstances, the TRL V5 concentration does not show a substantial response to pharmaceutical agent or fat loads, such that a response in "L TRL" may in particular circumstances track the TRL V6 response itself and can then be considered an equivalent thereof. This, however, does not hold true for measuring the total TRL or triglyceride concentration (or particle number) of the group as a whole (as for example total VLDL). It should be noted that the measure of the various TRL's may be reported as either concentration, ultimately meaning the concentration of triglycerides the designated TRL in the sample, or as the particle number, ultimately meaning the concentration of TRL particles in the sample. Both give the same results regarding the TRL V6 response or increase therein.

The term "VLDL size response" means the increase in particle size of TRL subclasses V1-6 as a group, in a patient or test mammal, in response to administration of a pharmaceutical agent or by fat load or high fat diet, which increase is the effect caused by the increase of TRL V6 concentration (or particle number) on the size distribution of VLDL particle sizes as a class (i.e. the change in the mean size of the TRL subpopulations, V1 - V6). As with L TRL, measuring VLDL size may under certain circumstances be used as a surrogate for measuring TRL V6 directly such that the VLDL size response can then be considered an equivalent of TRL V6 response.

An increase in TRL V6 response means a statistically significant increase in the measured TRL V6 concentration (or particle number or VLDL particle size). In one, example, an increase would be defined for a fasting assessment as a change in the TRL V6 response of approximately a 3.6 fold increase or 20-360% increase from pre-dose concentrations. An increase would be defined for assessment with a lipid load as a change in the TRL V6 response that falls above at least the 80^{th} percentile confidence interval of the upper bound of distribution of inter-occasion excursions measured in a population of patients in the absence of the pharmaceutical agent. For any given assay protocol, the actual confidence interval selected to determine a statistically significant response to the pharmaceutical agent will depend on the desired predictive value of the assay protocol. As for example, an assay protocol desiring to provide fewer false positive TRL V6 responses or increases in TRL V6 response will select a higher percentile confidence interval, say for example, the 90^{th} percentile, or for example, the 95^{th} percentile. For assay protocols desiring fewer false negatives, lower confidence intervals would be suitable, as for example the 80^{th} percentile.

The term "pharmaceutical agent" means a pharmaceutical active agent for which there has been an observed treatment emergent weight gain. In one example, pharmaceutical agent is taken to be an atypical antipsychotic pharmaceutical agent, as for example olanzapine, clozapine, quetiapine, ziprasidone, amisolpride, aripiprazole, asenapine, iloperidone, melperone, paliperidone, perospirone, risperidone, sertindole, sulpiride, and the like. In another example, the pharmaceutical agent is an antidepressant or mood stabilizer for which there has been the observation of treatment emergent weight gain, as for example amitriptyline, mirtazapine, lithium, valproic acid and carbamazepine. In another example, the pharmaceutical agent is a PPAR gamma agonist such as the class of thiazolidinedione compounds, as for example pioglitazone, rosiglitazone, or troglitazone. In yet another example, the pharmaceutical agent is an antiepileptic drug (AEDs) for which there has been the observation of treatment emergent weight gain, as for example valproate, carbamazepine and gabapentin.

The term "biosample" includes whole blood, plasma, serum, urine, cerebral spinal fluid (CSF), lymph samples, stool samples, tissues, and/or body fluids in raw form and/or in preparations. However, whole blood or plasma biosamples may be particularly suitable.

The term "substantial weight gain" means weight gain equal to or greater than about 5 lb (2.3 Kg) of body weight within the first month of treatment or weight gain equal to or greater than about 6 lb (2.8 Kg) of body weight within the six weeks of treatment.

Lipoproteins include a wide variety of particles found in plasma, serum, whole blood, and lymph, comprising various types and quantities of triglycerides, cholesterol, phospholipids, sphyngolipids, and proteins. These various particles permit the solublization of otherwise hydrophobic lipid molecules in blood and serve a variety of functions related to lipolysis, lipogenesis, and lipid transport between the gut, liver, muscle tissue and adipose tissue. In blood and/or plasma, lipoproteins have been classified in many ways, generally based on physical properties such as density or electrophoretic mobility. Classification based on nuclear magnetic resonance-determined particle size distinguishes at least 16 distinct triglyceride rich lipoprotein particle subtypes, including 5 subtypes of high density lipoproteins, 4 subtypes of low density lipoproteins, and 6 subtypes of very low density lipoproteins, designated TRL V1 through V6, and chylomicrons. Out of these lipoprotein subtypes, and in contrast to the other subtypes, the present invention has determined that the largest TRL particle subtype,TRL V6, can be used as a biomarker of triglyceride and/or lipoprotein metabolism in that concentrations of TRL V6, predictively become elevated following consumption of a fat-containing meal and then return to basal levels at some point subsequent to the meal as the test subject approaches a fasted state.

To correlate NMR characterizations of the TRL particles to estimated diameters, **Table 1** below defines the chemical shift for the TRL V6 range as well as for TRL V5 and chylomicrons.

**TABLE 1: Characteristics of Triglyceride Rich Lipoprotein Subclasses Measured by NMR LipoProfile® Analysis**

| Subclass | TRL Subclass Components | NMR Chemical Shift (ppm) | Estimated Diameter (nm) |
|---|---|---|---|
| Chylomicrons | C-260 | 0.8477 | 260 |
| Chylomicrons | C-250 | 0.8470 | 250 |
| Chylomicrons | C-240 | 0.8464 | 240 |
| Chylomicrons | C-225 | 0.8457 | 225 |
| Chylomicrons | C-200 | 0.8443 | 200 |
| Chylomicrons | C-190 | 0.8440 | 190 |
| Chylomicrons | C-185 | 0.8436 | 185 |
| Chylomicrons | C-180 | 0.8429 | 180 |
| Chylomicrons | C-175 | 0.8422 | 175 |
| Chylomicrons | C-170 | 0.8416 | 170 |
| TRL V6 | V6-140 | 0.8402 | 140 |
| TRL V6 | V6-120 | 0.8388 | 120 |
| TRL V6 | V6-100 | 0.8374 | 100 |
| TRL V5 | V5-80 | 0.8361 | 80 |
| TRL V5 | V5-70 | 0.8347 | 70 |
| TRL V5 | V5-60 | 0.8333 | 60 |

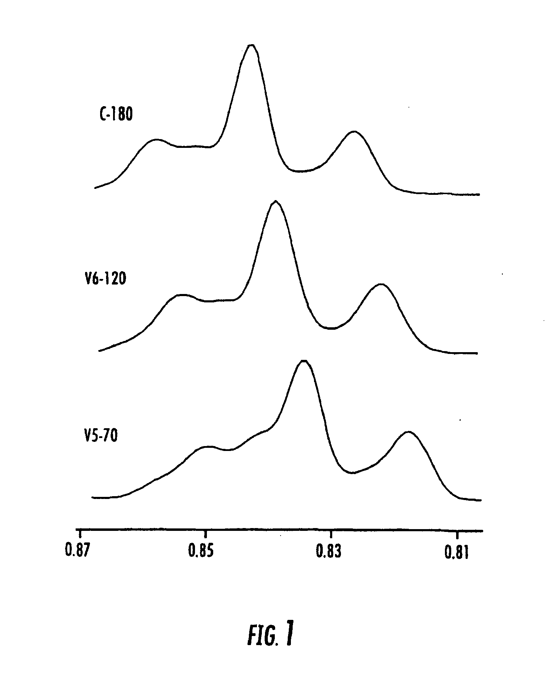

**Table 1** illustrates proton NMR chemical shifts of isolated triglyceride rich lipoprotein (TRL) subclasses (subfractions) that were measured relative to the internal reference signal of Ca EDTA (2.519 ppm). **Figure 1** illustrates NMR characteristic signals of exemplary TRL subclasses. NMR measurements were conducted on a 400 MHz spectrometer at 47°C. TRL subclasses identified as chylomicrons were isolated from postprandial plasma specimens obtained from human subjects after ingestion of a fat-containing meal. TRL subclasses identified as TRL V6 or TRL V5 were obtained from fasting plasma specimens obtained from hypertriglyceridemic human subjects. TRL subclasses were initially isolated by sequential ultracentrifugation (density <0.94 g/m for chylomicrons and <1.006 g/mL for TRL V5-V6) and further purified by gel filtration chromatography using 1% or 2% agarose beads (Bio-Rad, Hercules, CA) in a buffer containing 120 mM KCl, 5 mM EDTA, 1 mM CaCl₂, 50 mM Na₂HPO₄ and 0.2 g/L NaN₃, pH 7.4. Estimates of lipoprotein diameters were obtained from electron microscopy measurements on the isolated TRL subclasses.

Thus, while TRL V6 has been defined by diameter hereinabove, the NMR chemical shifts in **Table 1** represent equivalents to the defined diameter ranges. Thus, using NMR evaluation, TRL V6 can also be defined as having chemical shifts between about 0.8374 to about 0.8402 and the neighboring TRL subfractions (e.g., TRL V5 and chylomicrons) have the chemical shifts also noted in **Table** 1. Accordingly, the definition of "TRL V6" also refers to any TRL subfraction that has the NMR chemical shifts noted above (+/- reasonable measurement ranges) when measured as described even if the actual test at issue employs a non-NMR evaluation methodology or defines the parameter with respect to density or in another manner rather than diameter.

For example, one known technique to measure very large TRL particles is flotation ultracentrifugtion that employs a density-based separation. Redgrave et al. have characterized particles by their flotation rate (S_{f}, Svedberg units) with respect to their estimated diameters: S_{f} >400 includes particles >75 nm; S_{f} 175-400 includes particles between 50-75 nm; S_{f} 100-175 includes particles between 37-50 nm; and S_{f} 20-100 includes particles between 20-37 nm. *See*, Redgrave et al., Changes in plasma in very low density and low density lipoprotein content, composition, and size after a fatty meal in normo- and hypertriglyceridemic man, Journal of Lipid Research, Vol. 20, pp. 217-229 (1979). *See also,* Karpe et al., Differences in Postprandial Concentrations of Very-Low-Density Lipoprotein and Chylomicron Remnants Between Normotryglicyeridemic and Hypertriglyceridemic Men With and Without Coronary Heart Disease, Metabolism, Vol. 48, No. 3 (March), 1999, pp. 301-307. Thus, even if not characterized based on size by the test method itself, if the density separated particles have about the chemical shifts noted above, the TRL particles are TRL V6 particles.

Furthermore, the present specification has determined that the post prandial triglyceride level correlates with elevation of TRL V6 concentration. These triglyceride levels can be attributed to either intra-lumenal triglyceride being hydrolyzed, fatty acids being absorbed from the gut and then re-esterified as triglyceride and transported into the lymphatics or the portal circulation as chylomicrons, or to VLDL secreted by the liver. In a state of positive energy balance, as for example, after excessive food intake, triglycerides are transported as a component of VLDL to adipose tissue for storage, as opposed to or in addition to other tissues, such as muscle, for use for energy through fatty acid oxidation. Thus an increase in post prandial triglyceride levels would indicate a decrease in fatty acid oxidation and/or an increase in fat absorption and, in any case, correlates with an increase in lipid storage. The present disclosure demonstrates that specifically an increase in TRL V6 concentration correlates with this increase in lipid storage, such that determination of changes in the TRL V6 concentrations due to treatment with a pharmaceutical agent can be used as a biomarker for effects on triglyceride and/or lipoprotein metabolism, particularly as a marker for an increase in lipid storage, including predicting whether a given individual patient will gain substantial weight when treated with the pharmaceutical agent.

It will be understood that elevation of TRL V6 concentration (or particle number) may in some instances also be tracked by the increase in mean VLDL particle size, (mean or average size for all lipoprotein particles in the group of VLDL lipoprotein particles, TRL V1-V6) or even just large TRL = TRL V5 +TRL V6). This is because the dominant component showing a changing concentration in response to pharmacologic agent in this group is the TRL V6 component as described above. Such a signal will be diluted by the signal from the other subtypes, but it may be possible to discern a VLDL size response (change in ave. size) and thus an increase in a VLDL size response as a surrogate to measuring the TRL V6 response directly. It is noted that the overall concentration of VLDL V1-V5 as a group does not appreciably change and TRL V6 response is not generally detectable from measuring VLDL concentrations (as distinct from mean size) as a class.

For studies in humans, including investigational studies on test therapeutic agents and for testing an individual's risk for substantial treatment emergent weight gain during treatment with a given therapeutic agent, a human patient is typically tested after waking and prior to consuming anything, as for example after fasting for about 5 - 10 hours, preferably between about 6 to 9 hours, as for example about 8 hours. Patients may drink water at any time before or during the testing period. A blood sample is then taken to provide a basal level measure of TRL V6 concentration or particle number for that patient when the patient is in a fasted state. The human patient is then administered the pharmaceutical agent and a second blood sample is take. The timing of this blood sample will depend on the pharmacokinetics of the pharmaceutical molecule. Depending on the molecule, the blood samples could be collected as soon as a 1 hour post dose to several days post treatment. For short assessments, as for example within a couple of hours, the patient needs to remain fasting until the assessment is complete. Typically, one post-dosing blood draw will be sufficient for routine testing to determine if the patient is at risk of treatment emergent weight gain during treatment with the pharmaceutical agent. Alternatively, multiple post-dosing blood draws may be taken to obtain a complete TRL V6 response time course. It is common to see the TRL V6 response be increased by between about 20% to about 360%, or approximately 3.6 fold from baseline. Typical responses indicative of a response indicative of future treatment emergent weight gain may be about 50% increase or more.

As an alternative to testing under fasted conditions, a suitable fat load may be administered, to standardize the TRL V6 concentration during testing. In this alternative, a human patient is typically tested after waking and prior to consuming anything, as for example after fasting for about 5 - 10 hours, preferably between about 6 to 9 hours, as for example about 8 hours. Patients may drink water at any time before or during the testing period. The pretest period can be made to facilitate consistency as to meal content and timing prior to each testing session. A blood sample is then taken to provide a basal level measure of TRL V6 concentration or particle number for that patient when the patient is in a fasted state The patient is then administered a suitable fat load over the course of a relatively short time period, as for example, in about ≤ 40 minutes, preferably in ≤ 20 minutes. Depending on the study conducted or preferred routine testing conditions desired, one or more blood samples are then taken to determine the elevation in TRL V6 concentration in response to the fat load. Typically, one post-fat load blood draw will be sufficient for routine testing to determine if the patient is at risk of treatment emergent weight gain during treatment with the pharmaceutical agent. Alternatively, multiple post-fat load blood draws may be taken to obtain a complete TRL V6 response time course. The human TRL V6 response to a fat load, (i.e. elevation of TRL V6 concentration) typically begins about 2 hr. post fat load administration, peaking between about 4 hr. and about 7 hr. post fat load administration, and returning to basal concentrations between about 9 hr. and about 12 hr. post fat load administration. Note that there may be a shift in time course to later onset/later return to basal levels when very high fat concentrations are administered. Such a shift typically sees an onset at about 4-6 hr., peaking at about 10 hr., and returning to basal levels between about 12 - 15 hr. A typical human TRL V6 response to a fat load is an elevation in plasma concentration from a basal level of about 0 mg/dL to about 35 mg/dl at a fasted state to between about 80 and about 250 mg/dL.

The patient is then retested in conjunction with administration of the pharmaceutical agent. It is to be understood that the test in conjunction with the pharmaceutical agent may be conducted either before or after the test to determine the patient's normal TRL V6 response, provided that the pharmaceutical agent has cleared the patient's system prior to retesting, though this is not preferred. A typical increase in human TRL V6 response indicative of a response predictive of treatment emergent weight gain to the test pharmaceutical agent is a statistically significant increase in the TRL V6 response. It is common to see the TRL V6 response be increased by approximately 3.6 fold or between about 20% to about 80%. Typical responses indicative of a positively predictive response may be about 50% increase or more.

In one example, the routine use in determining a given patient's risk of treatment emergent weight gain during treatment with a given pharmaceutical agent, (i.e. to predict if the patient will gain substantial weight during treatment with the agent) a single administration of a fat load in conjunction with a dose of the pharmaceutical agent may be suitable, such that the increases in TRL V6 response may be determined from a predetermined patient population average TRL V6 response to the given fat load. In such an example, one blood draw prior to administration of the fat load and pharmaceutical agent and a single blood draw at a predetermined time point post fat load administration can be sufficient to determine if the pharmaceutical agent increases the patient's TRL V6 response to the fat load, and thus determine if the agent adversely modulates that individual patient's triglyceride and/or lipoprotein metabolism, indicating the patient is at risk of treatment emergent weight gain if treated with the pharmaceutical agent.

In a further optimized example, it may be possible to determine a relevant TRL V6 response to a pharmaceutical agent or increase in TRL V6 response to a fat load in response to a pharmaceutical agent without an initial blood draw (i.e. without a basal TRL V6 concentration/particle number determination for the individual patient). In such an example, the measured TRL V6 concentration or equivalent is measured from a single biosample taken within a predetermined time range post dosing or post administration of the fat load in conjunction with administration of the pharmaceutical agent, and is then compared to a standard baseline or basal level determined for the relevant population being tested or compared to a standard distribution of responses to a fat load in the absence of any pharmaceutical agent.

It is also contemplated that the administered fat load may be chemically and/or pharmaceutically induced or a simulated fat load rather than a food-based fat load.

It will be understood that the specific clinical assay protocols used will depend upon the pharmaceutical agent being considered for therapy and that the determination of such protocols is within the ordinary skill in the art. As for example, the particular timing of administration of the agent and the second fat load will vary depending on the pharmacokinetics of the agent. One factor to consider is that the agent's blood levels be at an effective level prior to the administration of the fat load. Thus "in conjunction with" the pharmaceutical agent is understood to mean that the administration of the agent and the fat load are coordinated to allow the agent to be at an effective level in the patient so as to affect the absorption/distribution/metabolism/elimination of the fat load when it is administered. This can mean, depending on the specifics of the agent, that the agent is administered at the same time as the fat load or shortly thereafter, up to about 30 minutes post fat load administration, or that the agent is administered prior to the fat load by the period necessary to achieve an effective plasma level of the agent, say for example between about 0 and about 3 hours prior to administration of the fat load, as for example between 15 minutes and 30 minutes prior to administration of the fat load. If the pharmaceutical agent is administered as a prodrug or if active metabolites are important in the pharmacodynamics of the pharmaceutical agent, a longer time may be necessary to allow for appropriate plasma concentrations of the active moieties to accumulate. Determination of such dosing regimens are routine and well within the ordinary skill of the art.

Similarly, the timing and number of blood draws after administration of the pharmaceutical agent, or fat load in conjunction with administration of the pharmaceutical agent, may vary depending on the clearance rate of the agent, the important factor being that the agent's blood levels remain at an effective level until after the post fat load blood draw and should optimally be timed to correspond to the peak TRL V6 response based on the pharmaceutical agent and/or fat load administered.

In examples where a fat load is used, suitable fat loads comprise equal or greater than about 30 g of fat, preferably greater than or equal to about 50 g of fat, preferably between about 60-70 g of fat. Fat loads comprising greater than or equal to about 80 g of fat may begin to induce longer times to TRL V6 response on set.

It will be understood that the exact composition of the fat load is unimportant and many suitable compositions may be readily formulated by the ordinarily skilled dietician. Suitable fat load formulations may be tailored to suit the desired study and/or according to patient population preferences. The important factor is that sufficient fat content and excessive over all caloric content be provided within a short time period to effect a measurable elevation in TRL V6 concentration in the absence of a test pharmaceutical agent.

The fat load may take any of a number of suitable forms as for example a prepared meal, a prepackaged meal, a single dose food stuff such as a snack bar, single dose liquid composition such as a prepackaged beverage or powdered beverage mix, or oral tablets or capsules. Examples of suitable compositions that would serve as fat loads are as follows:

### Fat load Example 1:

| Food Items | Portion | Energy (kcal) | Carb. (g) | Protein (g) | Fat (g) |
|---|---|---|---|---|---|
| Rice | 1 ice cream scoop (50 g) | 65 | 15 | 1 | 0 |
| Margarine (to be added into rice while it is hot) | 1 level dessertspoon (10g) | 72 | 0.0 | 0.0 | 8.0 |
| Fried chicken wing (flour coated) | 1 wing (70g) | 223 | 6.7 | 19.1 | 13.3 |
| Hash brown potatoes | 2 pieces (120g) | 378 | 33.0 | 2.9 | 26 |
| Grade-A eggs (fried) | 1 egg (35g) | 90 | 0.1 | 5.7 | 7.4 |
| Sliced cucumber | 5 slices (15g) | 2 | 0.5 | 0 | 0 |
| Total | | 830 | 55 | 27 | 55 |
| Total Calories | | 830 | 229 | 108 | 495 |
| % Calories | | | 28% | 13% | 60% |

### Fat load Example 2:

| Menu | Protein (g) | Fat (g) | Carb. (g) | Total CAL. | % Protein | % Fat | % Carb. |
|---|---|---|---|---|---|---|---|
| | | | | | | | |
| 1 two-egg omelet | 12 | 14 | 2 | 180 | | | |
| 30 mL peanut butter | 7 | 16 | 7 | 200 | | | |
| 2 slices light bread, toasted | 4 | 0.5 | 17 | 80 | | | |
| 237 mL whole milk | 8 | 8 | 12 | 150 | | | |
| 2 strips bacon | 5 | 7 | 0 | 80 | | | |
| 30 mL margarine | 0 | 7 | 0 | 66 | | | |
| Meal totals (served) | 36 | 52.5 | 38 | 756 | 18.7% | 61.5% | 19.8% |

Fat load Example 3: Beverage: Volume of whole milk or cream or mixture thereof to provide desired amount of fat. For example about 150 mL to about 180 mL of whipping cream. A flavoring agent may be added to improve palatability.]

### Measurement of TRL V6 concentration or particle number

TRL V6 response can be measured in any suitable manner. One currently known method of selectively measuring TRL V6 concentration or particle number is by NMR spectroscopy on plasma samples followed by deconvolution analysis to separate out the relative contributions of the various lipoprotein subtypes to the complete NMR signal. One such deconvolution method is the NMR LipoProfile^{®}, NMR LipoProfile^{®}-II and/or NMR LipoProfile® III, subclass particle analysis as provided by LipoScience, Inc., Raleigh, North Carolina. Likewise, meanVLDL particle size may be measured with the NMR LipoProfile^{®} subclass particle analysis as well. *See* US Pat. No. 5,343,389 to Otvos, US Pat. No. 6,617,167, US Patent No. 4,933,844, and US Patent No. 7,243,030, for a description of this analytical technique. *See also*, US patent application 2005-0222504 for a description of NMR Clinical Analyzers. *See also* Handbook of Lipoprotein Testing, Chapter 31: "Measurement of lipoprotein subclass profiles by nuclear magnetic resonance spectroscopy", J.D. Otvos, AACC Press, Washington DC, 2000, 2nd ed., pp 609-623, and Jeyarajah EJ, Cromwell WC, Otvos JD. Lipoprotein particle analysis by nuclear magnetic resonance spectroscopy. Clin Lab Med. 2006;26:847-70.

As a typical example, whole blood samples are collected into 2 ml EDTA blood collection tubes, inverted several times to mix well, and placed on ice. The samples are then centrifuged at approximately 3000 rpm for 10 - 15 minutes at 4°C. Upon completion of centrifugation, the tube is placed on ice and the plasma is drawn off. The plasma is place into a polypropylene tube and kept at 4°C until analysis. Samples may be analyzed at any time up to about 4 days after collection. Samples are analyzed for TRL V6 response, as for example by NMR plasma lipoprotein analysis, as for example, the NMR LipoProfile^{®}, the NMR LipoProfile^{®}-II or the NMR LipoProfile®-III lipoprotein tests provided by LipoScience, Inc. of Raleigh, North Carolina.

NMR spectral data is analyzed as described in Jeyarajah *et al*., *supra.* Lipoprotein subclass concentrations are reported in lipid mass concentration units (TRL subclasses in units of mg/dL triglyceride) or, alternatively, in particle concentration units of nmol/L (nanomoles of particles per liter). The source information (NMR signal amplitude of each subclass) is transformed into these mass or particle concentration units by the analysis software, using a set of conversion factors obtained from chemical lipid and NMR analyses of isolated chylomicron, VLDL, LDL, and HDL subclass standards of normal lipid composition.

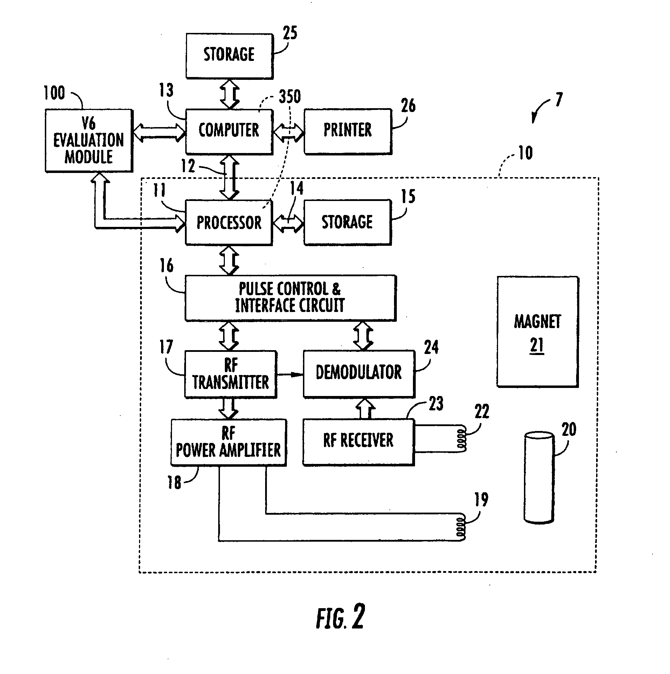

Figure 2 illustrates an exemplary NMR analyzer system 7 that can be used to carry out the TRL V6 analysis and/or TRL V6 measurements using a TRL V6 evaluation module **100.** The TRL V6 evaluation module **100** can be on-board the system in a signal processor and/or controller **11** or may reside partially or totally in a different local or remote processor such as on a server, client and/or other computer. Referring now to **Figure 2****,** the system 7 includes an NMR spectrometer **10** for taking NMR measurements of a biosample. In some embodiments, the spectrometer **10** is configured so that the NMR measurements are conducted at 400 MHz for proton signals; in other embodiments the measurements may be carried out at 360MHz or another desired frequency. That is, other frequencies corresponding to a desired operational field strength may also be employed. Typically, a proton flow probe is installed, as is a temperature controller to maintain the sample temperature at 47 +/- 0.2 degrees C. Field homogeneity of the spectrometer **10** can be optimized by shimming on a sample of 99.8% D₂O until the spectral linewidth of the HDO NMR signal is less than 0.6 Hz. The 90° RF excitation pulse width used for the D₂O measurement is typically ca. 6-7 microseconds.

Referring again to **Figure 2****,** the spectrometer **10** is controlled by a digital signal processor and/or controller **11** or other signal processing unit. The processor/controller **11** should be capable of performing rapid Fourier transformations and may include for this purpose a hard-wired sine table and hardwired multiply and divide circuit. It may also include a data link **12** to an external or remote computer **13,** and a direct-memory-access channel **14** which connects to an electronic storage unit **15**.

The processor/controller **11** may also include a set of analog-to-digital converters, digital-to-analog converters and slow device I/O ports which connect through a pulse control and interface circuit **16** to the operating elements of the spectrometer. These elements include an RF transmitter **17** which produces an RF excitation pulse of the duration, frequency and magnitude directed by the digital computer **11,** and an RF power amplifier **18** which amplifies the pulse and couples it to the RF transmit coil **19** that surrounds sample cell **20.** The NMR signal produced by the excited sample in the presence of a 9.4 Tesla polarizing magnetic field produced by superconducting magnet **21** is received by a coil **22** and applied to an RF receiver **23.** The amplified and filtered NMR signal is demodulated at **24** and the resulting quadrature signals are applied to the interface circuit **16** where they are digitized and input through the digital computer **11** to a file in the disc storage **15.** The system **7** can include a deconvolving module located in the signal processor/controller **11** and/or totally or partially in another processor on a different computer, server or client that may be on-site or remote. *See*, US2005/0222504 for additional description of suitable clinical NMR analyzers.

After the NMR data are acquired from the sample in the measurement cell **20,** signal processing produces a data file which is a digital representation of the chemical shift spectrum which may be stored in electronic archival medical record storage **25.** The computer **13,** which may be personal, laptop, desktop, or other computer, processes the chemical shift spectrum and can provide a patient report, which is output to a printer **26** or electronically stored and relayed to a desired email address or URL. Those skilled in this art will recognize that other output devices, such as a display, may also be employed to provide the results.

It should be apparent to those skilled in the art that the functions performed by the computer **13** and its storage **25** may also be incorporated into the functions performed by the spectrometer's digital signal processor/controller **11** or in additional circuits in communication with the NMR spectrometer **10** and/or processor **11.** Other interfaces and output devices may also be employed, as are well-known to those skilled in this art.

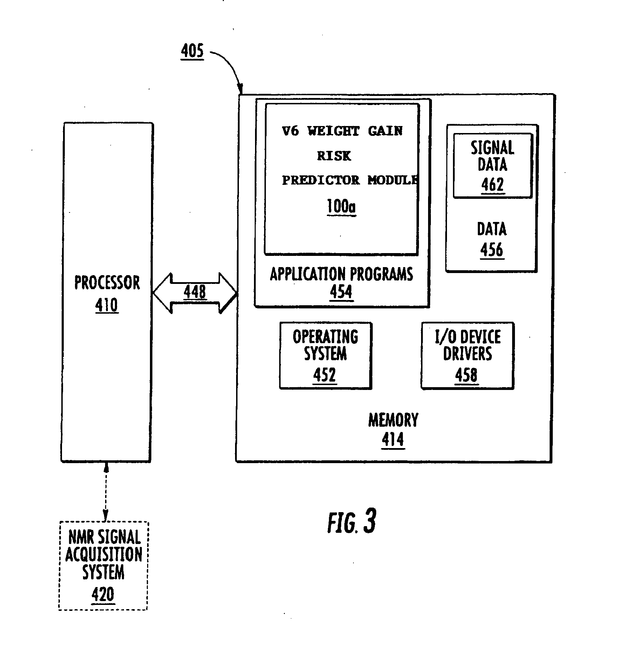

**Figure 3** illustrates an example of TRL V6 evaluation module **100a** that can communicate with system **7,** be on-board the system **7,** and or analyze TRL V6 data measured by the system **7** or other measurement systems that can provide the TRL V6 data. As shown, **Figure 3** illustrates an exemplary embodiment of data processing systems that can be incorporated or provided as systems, methods, and/or computer program products. The processor **410** (which can optionally be or communicate with processor **11** and/or computer **13** in **Figure 2****)** communicates with the memory 414 via an address/data bus **448.** The processor **410** can be any commercially available or custom microprocessor. The memory **414** is representative of the overall hierarchy of memory devices containing the software and data used to implement the functionality of the data processing system. **405.** The memory **414** can include, but is not limited to, the following types of devices: cache, ROM, PROM, EPROM, EEPROM, flash memory, SRAM, and DRAM.

As shown in **Figure 3**, the memory **414** may include several categories of software and data used in the data processing system **405:** the operating system **452;** the application programs **454;** the input/output (I/O) device drivers **458;** the TRL V6 Weight Gain Risk Predictor Module **100a (****Figure 3****);** and data **456.**

The data **456** may include TRL NMR subclass signal (constituent and/or composite spectrum lineshape) data **462** which may be obtained from a data or signal acquisition system **420** (such as system 7 shown in **Figure** 2). For each patient biosample, the data can include TRL V6 specific data values, or TRL V6 with other subfraction data (e.g., chylos and/or TRL V5). As will be appreciated by those of skill in the art, the operating system **452** may be any operating system suitable for use with a data processing system, such as OS/2, AIX or OS/390 from International Business Machines Corporation in Armonk, NY, Windows CE, Windows NT, Windows 95, Windows 98, Windows 2000, or Windows XP from Microsoft Corporation, Redmond, WA, Palm OS from PalmSource, Inc., Sunnyvale, CA, Mac OS from Apple Computer, Inc, UNIX, FreeBSD, or Linux, proprietary operating systems or dedicated operating systems, for example, for embedded data processing systems.

The I/O device drivers **458** typically include software routines accessed through the operation system **452** by the application programs **454** to communicate with devices such as I/O data port(s), data storage **456** and certain memory **414** components and/or the data acquisition system **420.** The application programs **454** are illustrative of the programs that implement the various features of the data processing system **405** and preferably include at least one application that supports operations according to embodiments of the present invention. Finally, the data **454** represents the static and dynamic data used by the application programs **454,** the operating system **452,** the I/O device drivers **458,** and other software programs that may reside in the memory **414.**

While examples of the present specification are illustrative, for example, with reference to the Module **100a,** being an application program in **Figure 3****,** as will be appreciated by those of skill in the art, other configurations may also be utilized. For example, the Module **100a** may also be incorporated into the operating system **452,** the I/O device drivers 458, or other such logical division of the data processing system **405.** Thus, examples should not be construed as limited to the configuration of **Figure 3****,** which is intended to encompass any configuration capable of carrying out the operations described herein. The Module **100a** may include computer program code for communicating with a remote control system (local or offsite).

### Example 1: Human Study with an atypical antipsychotic - olanzapine (Zyperxa®)

The literature reports that treatments with several atypical antipsychotics have observed treatment emergent weight gain during treatment in subpopulations of patients. (e.g. olanzapine, clozapine, quetiapine, ziprasidone, amisolpride, aripiprazole, asenapine, iloperidone, melperone, paliperidone, perospirone, risperidone, sertindole, and sulpiride, and the like.) To demonstrate the use of TRL V6 response to identify those patients at risk of treatment emergent weight gain, an exploratory biomarker study is conducted employing a single-center, randomized, open-label, parallel design, comparing metabolic changes observed upon administration of olanzapine (Zyprexa®) in healthy volunteers.

Prior to administration of olanzapine to subjects, a fasted pre-dose plasma sample is collected. Subjects are then given 5 mg of olanzapine daily for 2 days, followed by 10 mg oral olanzapine daily for the remainder of the study. Subjects are admitted to a clinic 4 days following treatment and a fasting blood sample is collected for the evaluation of a V6 response. Subsequent visits repeated the same procedures and are scheduled for every 8-11 days of treatment. Subjects are on therapy for at least 3 weeks, up to a maximum of 26 days. During each V6 evaluation period, subject weight is recorded. Blood samples are collected into 2 ml EDTA blood collection tubes, inverted several times to mix well, and placed on ice. The samples are then centrifuged at approximately 3000 rpm for 10-15 minutes at 4°C. Upon completion of centrifugation, the tube is placed on ice and the plasma is drawn off. The plasma is place into a polypropylene tube and kept at 4°C until analysis. Samples may be analyzed at any time up to about 4 days after collection. Samples are analyzed for TRL V6 response, as for example by NMR plasma lipoprotein analysis, as for example, the NMR LipoProfile^{®}, the NMR LipoProfile^{®}-II or the NMR LipoProfile®-III lipoprotein tests provided by LipoScience, Inc. of Raleigh, North Carolina.

TRL V1, V2, V3, V4, V5 and V6 responses and VLDL size response are measured during each visit along with increases in weight. The olanazapine treatment on average show a statistically significant increase in the TRL V6 response of about 63%. The relationship of the change in TRL V6 to the change in weight is analyzed using a mixed effects model analysis. A statistically significant relationship is found between the individual change in TRL V6 and the change in weight. The relationship of the change in concentrations of any of the TRLs: V1, V2, V3, V4, or V5 subtypes to weight is not found to be significant. VLDL size is also significant, although the slope of this relationship is found to be less than the change in TRL V6 response. In most subjects, the administration of olanzapine increases the magnitude of the TRL V6 response and VLDL size response comparison to pre-dose concentration. A response predictive of treatment emergent weight gain in this study is approximately 360% increase or 3.6 fold increase with a change of 6.5mg/dl from baseline with 90% CI of (3.77, 9.13) while subject with less risk has a 1.1 fold increase with a change 0.38mg/dl from baseline with a 90% CI or (-0.24, 2.0). Thus the increase of the TRL V6 response and VLDL size response induced by the olanzapine correlates with the observed atypical antipsychotic treatment emergent weight gain in humans.

### Statistical analysis of the relationship between the change in weight to change in variable from baseline in healthy volunteers receiving Zyprexa

| Variable | Slope | P-Value |
|---|---|---|
| Chylos | -0.02351 | 0.839 |
| TRL V1 | 0.1044 | 0.736 |
| TRL V2 | -0.08345 | 0.248 |
| TRL V3 | -0.07896 | 0.180 |
| TRL V4 | -0.00949 | 0.390 |
| TRL V5 | 0.007301 | 0.505 |
| TRL V6 | 0.1652 | 0.050 |
| VLDL size | 0.08075 | 0.001 |
| V5 + V6 | 0.04860 | 0.181 |
| V5 + V6 + Chylos | 0.04521 | 0.188 |
| NTG | -0.00093 | 0.879 |

## Claims

1. A method of determining a human patient's risk of treatment emergent weight gain during treatment with a pharmaceutical agent comprising determining in a biosample isolated from the patient whether there is an increase in large triglyceride rich lipoprotein particles of the V6 subclass (TRL V6) concentration in response to one or more doses of the pharmaceutical agent, wherein a significant increase in TRL V6 concentration indicates the patient is at risk of treatment emergent weight gain during treatment with the pharmaceutical agent.

2. A method of determining a human patient's risk of treatment emergent weight gain during treatment with a pharmaceutical agent comprising:
measuring the TRL V6 response to one or more doses of the pharmaceutical agent in a biosample isolated from the patient administered said one or more doses of the pharmaceutical agent;
wherein a significant TRL V6 response indicates the patient is at risk of treatment emergent weight gain during treatment with the pharmaceutical agent.

3. A method of determining a human patient's risk of treatment emergent weight gain during treatment with a pharmaceutical agent comprising the steps of:
1) measuring the TRL V6 response to a first fat load in a biosample isolated from the patient administered the first fat load;
2) measuring the TRL V6 response to a second fat load in a biosample isolated from the patient administered a dose of the pharmaceutical agent in conjunction with administration of the second fat load;
3) determining whether there is an increase in TRL V6 response to the second fat load compared to the TRL V6 response to the first fat load;
wherein a significant increase in TRL V6 response indicates the patient is at risk of treatment emergent weight gain during treatment with the pharmaceutical agent.

4. A method for determining a human patient's risk of treatment emergent weight gain during treatment with a pharmaceutical agent comprising the steps of:
1) measuring the TRL V6 response to a fat load in a biosample isolated from the patient administered a dose of the pharmaceutical agent in conjunction with administration of the fat load;
2) determining whether there is an increase in TRL V6 response to the fat load compared to a standard TRL V6 response to the fat load absent the pharmaceutical agent;
wherein a significant increase in TRL V6 response indicates the patient is at risk of treatment emergent weight gain during treatment with the pharmaceutical agent.

5. The method according to any of Claims 1 to 4 wherein the pharmaceutical agent is an atypical antipsychotic drug.

6. The method according to Claim 5 wherein the pharmaceutical agent is olanzapine or salt thereof.

7. The method according to any one of Claims 1-6 wherein TRL V6 response is measured by NMR spectroscopy.

## Patentansprüche

1. Verfahren zur Bestimmung des Risikos eines menschlichen Patienten für eine Behandlung einer aufkommenden Gewichtszunahme während einer Behandlung mit einem pharmazeutischen Mittel, wobei das Verfahren ein Bestimmen in einer biologischen Probe, die aus dem Patienten isoliert wurde, daraufhin umfasst, ob es eine Erhöhung der Konzentration großer triglyceridreicher Lipoproteinteilchen der V6-Unterklasse (TRL-V6) in Reaktion auf eine oder mehrere Dosen des pharmazeutischen Mittels gibt, wobei eine signifikante Erhöhung der TRL-V6-Konzentration anzeigt, dass bei dem Patienten das Risiko für eine Behandlung einer aufkommenden Gewichtszunahme während einer Behandlung mit dem pharmazeutischen Mittel besteht.

2. Verfahren zur Bestimmung des Risikos eines menschlichen Patienten für eine Behandlung einer aufkommenden Gewichtszunahme während einer Behandlung mit einem pharmazeutischen Mittel, wobei das Verfahren umfasst:
Messen der TRL-V6-Reaktion auf eine oder mehrere Dosen des pharmazeutischen Mittels in einer biologischen Probe, die aus dem Patienten isoliert wurde, dem eine oder mehrere Dosen des pharmazeutischen Mittels verabreicht wurden;
wobei eine signifikante TRL-V6-Reaktion anzeigt, dass bei dem Patienten das Risiko für eine Behandlung einer aufkommenden Gewichtszunahme während einer Behandlung mit dem pharmazeutischen Mittel besteht.

3. Verfahren zur Bestimmung des Risikos eines menschlichen Patienten für eine Behandlung einer aufkommenden Gewichtszunahme während einer Behandlung mit einem pharmazeutischen Mittel, wobei das Verfahren die folgenden Schritte umfasst:
1) Messen der TRL-V6-Reaktion auf eine erste Fettbelastung in einer biologischen Probe, die aus dem Patienten isoliert wurde, dem die erste Fettbelastung verabreicht wurde;
2) Messen der TRL-V6-Reaktion auf eine zweite Fettbelastung in einer biologischen Probe, die aus dem Patienten isoliert wurde, dem eine Dosis des pharmazeutischen Mittels in Verbindung mit einer Verabreichung der zweiten Fettbelastung verabreicht wurde;
3) Bestimmen, ob es eine Erhöhung der TRL-V6-Reaktion auf die zweite Fettbelastung im Vergleich zu der TRL-V6-Reaktion auf die erste Fettbelastung gibt;
wobei eine signifikante Erhöhung der TRL-V6-Reaktion anzeigt, dass bei dem Patienten das Risiko für eine Behandlung einer aufkommenden Gewichtszunahme während einer Behandlung mit dem pharmazeutischen Mittel besteht.

4. Verfahren zur Bestimmung des Risikos eines menschlichen Patienten für eine Behandlung einer aufkommenden Gewichtszunahme während einer Behandlung mit einem pharmazeutischen Mittel, wobei das Verfahren die folgenden Schritte umfasst:
1) Messen der TRL-V6-Reaktion auf eine Fettbelastung in einer biologischen Probe, die aus dem Patienten isoliert wurde, dem eine Dosis des pharmazeutischen Mittels in Verbindung mit einer Verabreichung der Fettbelastung verabreicht wurde;
2) Bestimmen, ob es eine Erhöhung der TRL-V6-Reaktion auf die Fettbelastung im Vergleich zu einer Standard TRL-V6-Reaktion auf die Fettbelastung in Abwesenheit des pharmazeutischen Mittels gibt,
wobei eine signifikante Erhöhung der TRL-V6-Reaktion anzeigt, dass bei dem Patienten das Risiko für eine Behandlung einer aufkommenden Gewichtszunahme während einer Behandlung mit dem pharmazeutischen Mittel besteht.

5. Verfahren nach einem der Ansprüche 1 bis 4, wobei das pharmazeutische Mittel ein atypisches Antipsychotikum ist.

6. Verfahren nach Anspruch 5, wobei das pharmazeutische Mittel Olanzapin oder ein Salz hiervon ist.

7. Verfahren nach einem der Ansprüche 1 bis 6, wobei die TRL-V6-Reaktion mittels NMR-Spektroskopie gemessen wird.

## Revendications

1. Procédé de détermination du risque chez un patient humain de prise de poids associée à un traitement au cours d'un traitement par un agent pharmaceutique comprenant le fait de déterminer dans un échantillon biologique isolé du patient s'il se produit une augmentation de la concentration des grosses particules de lipoprotéines riches en triglycérides de la sous-classe V6 (TRL V6) en réponse à une ou plusieurs doses de l'agent pharmaceutique, dans lequel une augmentation significative de la concentration en TRL V6 indique que le patient présente un risque de prise de poids associée à un traitement au cours d'un traitement par l'agent pharmaceutique.

2. Procédé de détermination du risque chez un patient humain de prise de poids associée à un traitement au cours d'un traitement par un agent pharmaceutique comprenant :
la mesure de la réponse des TRL V6 à une ou plusieurs doses de l'agent pharmaceutique dans un échantillon biologique isolé du patient auquel ont été administrées une ou plusieurs doses de l'agent pharmaceutique ;
dans lequel une réponse significative des TRL V6 indique que le patient présente un risque de prise de poids associée à un traitement au cours d'un traitement par l'agent pharmaceutique.

3. Procédé de détermination du risque chez un patient humain de prise de poids associée à un traitement au cours d'un traitement par un agent pharmaceutique comprenant les étapes permettant de :
1) mesurer la réponse des TRL V6 à une première charge graisseuse dans un échantillon biologique isolé du patient auquel a été administrée une première charge graisseuse ;
2) mesurer la réponse des TRL V6 à une deuxième charge graisseuse dans un échantillon biologique isolé du patient auquel a été administrée une dose de l'agent pharmaceutique conjointement avec l'administration de la deuxième charge graisseuse ;
3) déterminer s'il se produit une augmentation de la réponse des TRL V6 à la deuxième charge graisseuse par comparaison avec la réponse des TRL V6 à la première charge graisseuse ;
dans lequel une augmentation significative de la réponse des TRL V6 indique que le patient présente un risque de prise de poids associée à un traitement au cours d'un traitement par l'agent pharmaceutique.

4. Procédé de détermination du risque chez un patient humain de prise de poids associée à un traitement au cours d'un traitement par un agent pharmaceutique comprenant les étapes permettant de :
1) mesurer la réponse des TRL V6 à une charge graisseuse dans un échantillon biologique isolé du patient auquel a été administrée une dose de l'agent pharmaceutique conjointement avec l'administration de la charge graisseuse ;
2) déterminer s'il se produit une augmentation de la réponse des TRL V6 à la charge graisseuse par comparaison avec une réponse des TRL V6 standard à la charge graisseuse absente de l'agent pharmaceutique ;
dans lequel une augmentation significative de la réponse des TRL V6 indique que le patient présente un risque de prise de poids associée à un traitement au cours d'un traitement par l'agent pharmaceutique.

5. Procédé selon l'une quelconque des revendications 1 à 4, dans lequel l'agent pharmaceutique est un antipsychotique atypique.

6. Procédé selon la revendication 5, dans lequel l'agent pharmaceutique est l'olanzapine ou un sel de celle-ci.

7. Procédé selon l'une quelconque des revendications 1 à 6, dans lequel la réponse des TRL V6 est mesurée par spectroscopie par résonance magnétique.
